(19) Europäisches Patentamt
European Patent Office
Office européen des brevets

(11) **EP 3 237 033 B1**

(12) **EUROPEAN PATENT SPECIFICATION**

(45) Date of publication and mention
of the grant of the patent:
**05.08.2020 Bulletin 2020/32**

(51) Int Cl.:
***A61M 1/12*** *(2006.01)*          ***A61M 1/10*** *(2006.01)*
***A61F 2/24*** *(2006.01)*

(21) Application number: **14820874.7**

(86) International application number:
**PCT/EP2014/079096**

(22) Date of filing: **22.12.2014**

(87) International publication number:
**WO 2016/101991 (30.06.2016 Gazette 2016/26)**

(54) **AN IMPLANTABLE HYDRAULIC DISPLACEMENT ACTUATOR AND SYSTEM.**

IMPLANTIERBARER HYDRAULISCHER VERSCHIEBUNGSAKTUATOR UND SYSTEM.

ACTIONNEUR DE DÉPLACEMENT HYDRAULIQUE IMPLANTABLE ET SYSTÈME.

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.11.2017 Bulletin 2017/44**

(73) Proprietor: **Syntach AG**
**8200 Schaffhausen (CH)**

(72) Inventors:
• **SOLEM, Jan Otto**
**237 42 Bjärred (SE)**
• **ENGVALL, Daniel**
**302 93 Halmstad (SE)**

• **KRÜGER, Victoria**
**283 43 Oxie (SE)**
• **STRÖMSTEN, Patrick**
**435 35 Mölnlycke (SE)**

(74) Representative: **KIPA AB**
**P O Box 1065**
**251 10 Helsingborg (SE)**

(56) References cited:
**WO-A1-00/66196          US-A- 3 965 897**
**US-A1- 2002 188 170          US-A1- 2004 153 067**
**US-A1- 2006 178 550          US-A1- 2009 060 764**
**US-A1- 2012 083 825          US-A1- 2013 289 717**

## Description

BACKGROUND OF THE INVENTION

Field of the Invention

This invention pertains in general to the field of hydraulic displacement actuators and systems. More specifically, the invention relates to a hydraulic displacement actuator for moving a mitral and or tricuspid heart valve plane, also called the AV (atrio-ventricular) heart valve planes.

Description of the Prior Art

Currently hydraulic actuators have a seal which is more or less cylindrical arranged around the piston at the end of the housing of the actuator. The seal is arranged in such a manner for preventing leakage of oil from inside the housing to the outside. The oil is used for transferring energy to a mechanical movement. These actuators are generally quite large and have seals that wear out and need to be replaced. This replacement leads to unwanted stop and delays in the use of the machines and require the machines to be disassembled in order to replace the seal and other parts that have worn out. If the seal is not replaced there will be a leakage at the seal and the machines will perform badly or not at all, hence putting the machine in a static state wherein the machine cannot move any more.

US 2004/153067 A1 discloses an implantable hydraulic displacement actuator comprising a biocompatible hydraulic displacement fluid for providing a force inside a human or animal body.

US 2006/178550 A1 discloses an implantable hydraulic displacement actuator for providing a force inside a human or animal body comprising first and second fixation means for anchoring the hydraulic displacement actuator at a heart ventricle wall.

Hence, an improved hydraulic actuator overcoming the above drawbacks would be advantageous.

Summary of the invention

The invention is defined by claim 1. Preferred embodiments of the invention are defined by the dependent claims. Accordingly, examples of the present disclosure preferably seek to mitigate, alleviate or eliminate one or more deficiencies, disadvantages or issues in the art, such as the above-identified, singly or in any combination by providing a hydraulic displacement actuator, systems, manufacturing and exemplary methods thereof.

According to a first aspect of the disclosure an implantable hydraulic displacement actuator comprising a biocompatible hydraulic displacement fluid for providing a force inside a human or animal body, wherein said hydraulic displacement actuator preferably is a linear hydraulic actuator for generating a cardiac movement.

According to a second aspect of the disclosure a hydraulic displacement actuator comprising a housing, comprising a first end and a second end, a piston, comprising a first end and an extending section, wherein the first end of the piston is arranged in the housing and the extending section is arranged at least partly outside the housing, a sealing member, comprising a first end and a second end. And wherein the first end of the sealing member is secured to the second end of the housing and the second end of the sealing member extends outwards or inwards, along the piston, from a terminal edge at the second end of the housing, for sealing an interior space of the housing.

According to a third aspect of the disclosure a system for moving an implantable hydraulic displacement actuator comprising a hydraulic displacement actuator, comprising, a housing, comprising a first end and a second end, a piston, comprising a first end and an extending section, wherein the first end of the piston is arranged in the housing and the extending section is arranged at least partly outside the housing, a biocompatible hydraulic displacement fluid for pressurizing the hydraulic displacement actuator. The system also comprises a pressurizing means for pressurizing the biocompatible hydraulic displacement fluid and the hydraulic displacement actuator.

According to a fourth aspect of the disclosure an exemplary method of displacing a hydraulic displacement actuator comprising providing a hydraulic displacement actuator which comprises a housing, comprising a first end and a second end, a piston, comprising a first end and an extending section, wherein the first end of the piston is arranged in the housing and the extending section is arranged at least partly outside the housing, a biocompatible hydraulic displacement fluid for pressurizing the hydraulic displacement actuator. And, moving the housing in a first direction by pressurizing a space, comprising the biocompatible hydraulic displacement fluid, arranged between the first end of the housing and the first end of the piston. Or moving the housing in an opposite direction from the first direction by pressurizing a space, comprising the biocompatible hydraulic displacement fluid, arranged between the second end of the housing and the first end of the piston.

According to a fifth aspect of the disclosure an exemplary method of implanting a hydraulic displacement actuator comprising providing a guide wire to a desired location, and inserting the hydraulic displacement actuator to a desired position in a heart over the guide wire.

According to a sixth aspect of the disclosure a non-transitory medium has instructions for 3D-printing a hydraulic actuator or system comprising a hydraulic actuator.

Some examples of the disclosure provide for no risk of damaging the body of the human or animal.

Some examples of the disclosure provide for a biocompatible fluid which will hinder any negative reaction between the biocompatible fluid and other fluids in the body and thus no damage to the actuator or body.

Some examples of the disclosure provide for a greatly reduced risk of leakage from a housing out into surroundings or into the housing.

Some examples of the disclosure provide for an AV valve or valve plane, e.g. mitral valve and/or mitral valve plane being moved with the movement of an actuator.

Some examples of the disclosure provide for fixation means configured to be detachable from an actuator if the actuator has to be changed while the fixation means remain attached.

Some examples of the disclosure provide for fixation means being configured to be fixated at or close to an apex of a heart or a heart ventricle, e.g inside a heart muscle, inside a heart muscle wall or on an outer surface of the heart. Or, the fixation means being configured to be attached in adjacent non heart structures like the thoracic wall or the columna vertebrae (the spine).

Some examples of the disclosure provide for a displacement actuator anchored at a distance which allows for the actuator to have enough stroke length to move an AV valve or valve plane, e.g. mitral valve and/or mitral valve plane a desired and/or required distance such that the pumping action of the heart is restored to normal function or improved close to normal function.

Some examples of the disclosure provide for an attachment element that is easily secured to different objects, such as an anchor.

Some examples of the disclosure provide for an attachment element having releasable attachment mechanism for detaching the displacement actuator if it has to be exchanged or removed.

Some examples of the disclosure provide for an hydraulic actuator wherein a housing of the hydraulic actuator is moving while a piston of the hydraulic actuator is stationary.

Some examples of the disclosure provide for displacement actuator which is a double acting actuator.

Some examples of the disclosure provide for an hydraulic actuator producing two forces which are applied to the mitral or tricuspid valve and/or mitral valve plane such that they move away or towards the apex of the heart.

Some examples of the disclosure provide for forces being produced preferably along the long axis of the heart ventricle, for assisting the pumping function of the heart.

Some examples of the disclosure provide for an hydraulic actuator being a single acting actuator, thus producing a force in only one direction, on outstroke or on instroke.

Some examples of the disclosure provide for a force moving the AV valve or AV plane such as the mitral valve and/or mitral valve plane away and/or toward the apex of the heart such that the normal pumping function of the heart is assisted.

Some examples of the disclosure provide for an hydraulic actuator which is is sealed off from its surroundings and thus creates an improved complete seal.

Some examples of the disclosure provide for a reduced friction between a piston and any tissue surrounding it.

Some examples of the disclosure provide for a sealing member which prevents any adhesion of tissue, body fluids such as blood or the alike because it will be act as a protective barrier.

Some examples of the disclosure provide for a sealing member adapted to withstand repeated movements of expansion and compression due to its elasticity and flexibility.

Some examples of the disclosure provide for a sealing member which has a spring effect for assisting a piston to move in the outstroke and/or instroke motion of the piston.

Some examples of the disclosure provide for a sealing member which is easily constructed by separate components.

Some examples of the disclosure provide for a hydraulic displacement actuator comprising means for disengaging such that substantially no force is produced by the displacement actuator.

Some examples of the disclosure provide for a displacement actuator which is free to move with a beating heart.

Some examples of the disclosure provide for a displacement actuator which is adapted to account for a failure.

Some examples of the disclosure provide for a displacement actuator without at least partly affecting the heart and possible damaging the heart.

Some examples of the disclosure provide for a displacement actuator wherein a biocompatible hydraulic fluid can freely flow.

Some examples of the disclosure provide for a displacement actuator configured to have a reduced energy consumption.

Some examples of the disclosure provide for a hydraulic actuator being guided by a guide wire extended through the hydraulic actuator.

Some examples of the disclosure provide for a displacement actuator which can be kept small and compact.

Some examples of the disclosure provide for a displacement actuator which is streamlined for a least flow resistance.

Some examples of the disclosure provide for a displacement actuator being streamlined such that any disturbance to the natural flow of the blood in a heart when passing the displacement actuator is reduced.

Some examples of the disclosure provide for a displacement actuator which prohibits any substances to easily get caught on selected parts of the displacement actuator and lead to complications within the heart such as clothing, tissue ingrowth or other unwanted cardiac complications.

Some examples of the disclosure provide for a displacement actuator to easily be transferred through a catheter.

Some examples of the disclosure provide for a displacement actuator comprising a piston which is stationary and while a housing 10 is moving when the displacement actuator is acting.

Some examples of the disclosure provide for a displacement actuator being controllably moved and the force by which the displacement actuator is moved by a pressurizing means.

Some examples of the disclosure provide for a control unit which is configured to control a hydraulic actuator based on sensor information from at least one sensor.

Some examples of the disclosure provide for a control unit which controls a pressurizing means by use of an electrical signal of the heart e.g. the beat of the heart.

Some examples of the disclosure provide for a control unit which controls a pressurizing means by use of a pressure signal inside the heart e.g. left ventricular or atrial pressure.

Some examples of the disclosure provide for a control unit which control a pressurizing means by use of a position signal.

Some examples of the disclosure provide for a control unit which controls a pressurizing means by use of an acceleration signal of the heart e.g. the beat of the heart.

Some examples of the disclosure provide for a displacement actuator operating in synchronicity with a heartbeat and/or cycle.

Some examples of the disclosure provide for a control unit which controls a pressurizing means based on at least one sensor adapted to detect volumes, in the whole or parts of the actuator system.

Some examples of the disclosure provide for a displacement actuator which is refillable of a biocompatible hydraulic fluid.

Some examples of the disclosure provide for a displacement actuator printed by a 3D printer.

Some examples of the disclosure provide for a displacement actuator being tailored to a patient's bio data.

Further, the applicant have found that an implantable hydraulic actuator, in particular when embodied as a linear hydraulic actuator for providing a linear/substantially straight displacement/movement, has some surprising effects of small size, great effect compared to size, a low power consumption, a god movement velocity, less complicated technical solution and so on compared to other linear actuators such as electromagnet based, permanent magnet based, Nitinol motor, Piezo motor, rotating motor with linear gear and/or linear motor.

The applicant have also found out that the produced force of the implantable hydraulic actuator can be beneficially used inside a human or animal body at or adjacent to an AV plane valve or valve annulus or valve plane e.g a mitral heart valve or a tricuspid heart valve. The rationale for applying such force at an AV plane valve or valve ring is based on the importance of a longitudinal natural movement of such valves or valve planes along a long axis directed from such valves towards the apex of a heart. 2/3 of a hearts pump function originate from such longitudinal movement of AV valves or planes, since the valves function as pistons, accelerating and augmenting the blood stream towards the apex of the heart inside the ventricles. In diseased hearts, for instance in cases of heart failure, such movements are impaired, in most cases of chronic heart failure, the movement is only half of the normal circa 25 mm stroke.

BRIEF DESCRIPTION OF THE DRAWINGS

These and other aspects, features and advantages of which examples of the disclosure are capable of will be apparent and elucidated from the following description of examples of the present disclosure, reference being made to the accompanying drawings, in which

Fig. 1 is a schematic view of a hydraulic displacement actuator according to an example of the invention.
Fig. 2 is a schematic cross section of a hydraulic displacement actuator according to an example of the invention.
Fig. 3 is a schematic cross section of a hydraulic displacement actuator according to an example of the invention.
Fig. 4 is a schematic cross section of a hydraulic displacement actuator according to an example of the invention.
Fig. 5 is a schematic side view of an inlet and outlet element of a hydraulic displacement actuator according to an example of the invention.
Fig. 6 is a schematic side view of an end of a housing and an end of a piston of a hydraulic displacement actuator according to an example of the invention.
Fig. 7 is a schematic side view of an attachment element of a hydraulic displacement actuator according to an example of the invention.
Fig. 8 is an illustration of a system comprising a hydraulic displacement actuator and pressuring means according to an example of the invention.

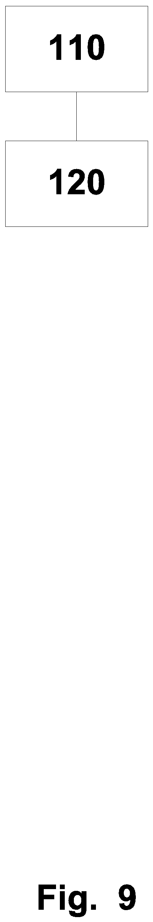

Fig. 9 is a flowchart of a method for moving a hydraulic displacement actuator according to an example of the disclosure.

DESCRIPTION OF THE PREFERRED EXAMPLES

Specific examples of the disclosure will now be described with reference to the accompanying drawings. This invention may, however, be embodied in many different forms and should not be construed as limited to the examples set forth herein. Rather, these examples are provided so that this disclosure will be thorough and complete, and will fully convey the scope of the invention, as claimed, to those skilled in the art. The terminology used in the detailed description of the examples illustrated in the accompanying drawings is not intended to be limiting of the invention. In the drawings, like numbers refer to like elements.

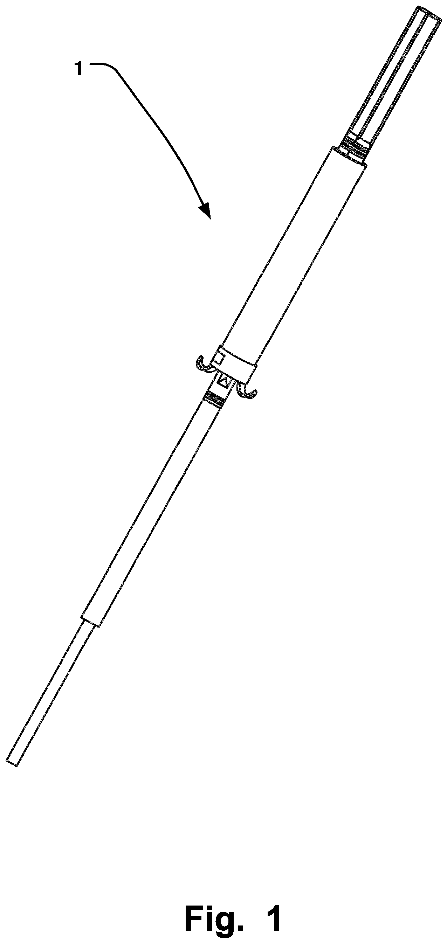

In an example, as e.g. illustrated in fig.1, is an implantable hydraulic displacement actuator 1, which comprises a biocompatible hydraulic displacement fluid for providing a force inside a human or animal body. Preferably the hydraulic actuator is a linear hydraulic actuator for generating a cardiac movement. By having the implantable hydraulic displacement actuator 1 comprising the biocompatible hydraulic displacement fluid, any risk of damaging the body of the human or animal is removed. Since, if the actuator would leak displacement fluid which is biocompatible the animal or human body will not be harmed by the leaking fluid. Additionally, the use of the biocompatible fluid will hinder any negative reaction between the biocompatible fluid and other fluids in the body and thus no damage to the actuator 1 can happen if body fluids enter the device. In an example the displacement fluid is a liquid chosen from a list comprising plain sterile water, sterile water with salts or electrolytes like saline or compositions of electrolytes like a Ringers solution and human plasma. Another example of the displacement fluid is a gas chosen from a list comprising helium, carbondioxid, Nitrogen or Hydrogen. Medical biocompatible fluids like hyaluron acids, hyaluron, iohexol, carboxymethyl-cellulose, hydroxypropylcellulose and dextrans may also be used. In an example a gas and a liquid is mixed.

In an example, the hydraulic displacement actuator comprises a sealing member 30 for sealing an interior space of the housing, which is further discussed below.

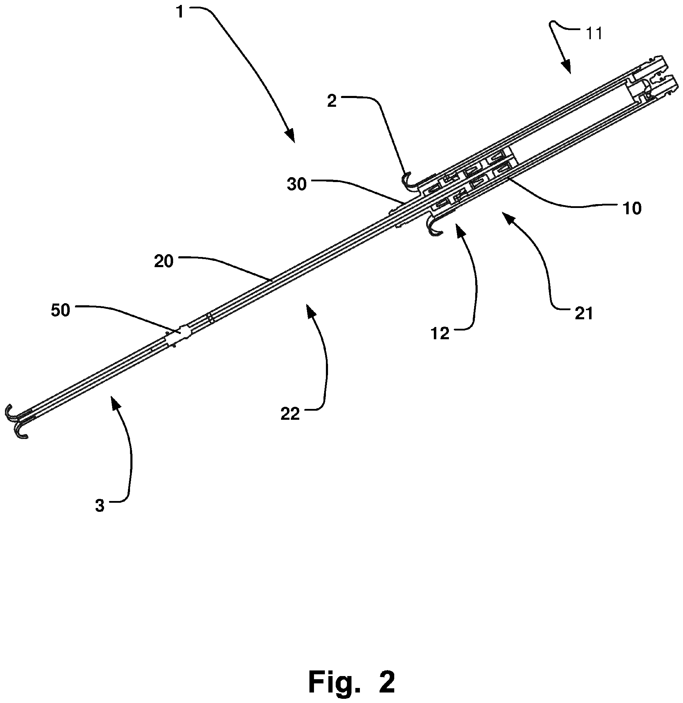

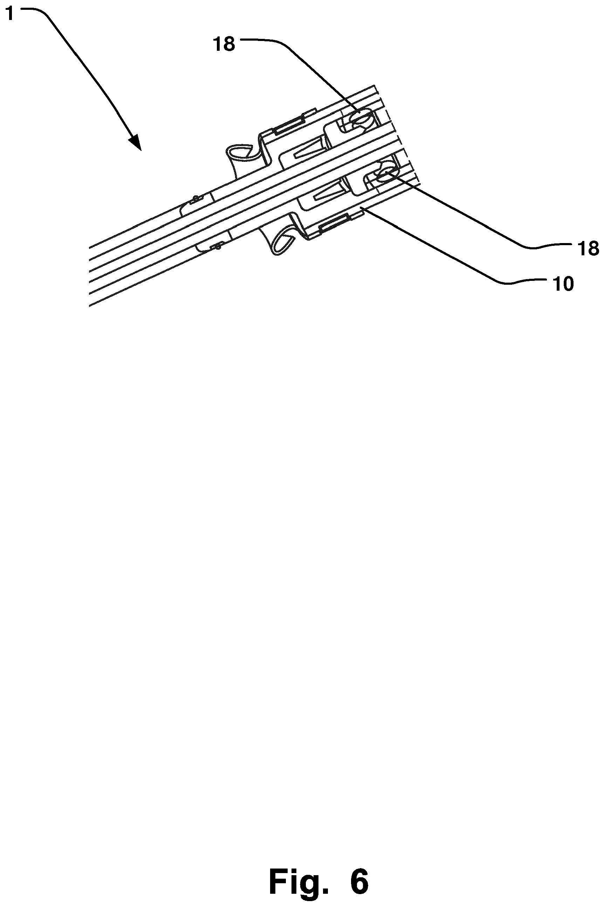

In an example, a hydraulic displacement actuator 1, as illustrated in e.g. fig.2-8, comprises a housing 10 and the housing 10 comprises a first 11 end and a second end 12. The hydraulic displacement actuator 1 comprises a piston 20 and the piston 20 comprises a first end 21 and an extending section 22. The first end 21 of the piston 20 is arranged in the housing 10 and the extending section 22 is arranged at least partly outside the housing 10. The hydraulic displacement actuator 1 comprises a sealing member 30 which comprises a first end 31 and a second end 32, and wherein the first end 31 of the sealing member 30 is secured to the second end 12 of the housing 10 and the second end 32 of the sealing member 30 extends outwards or inwards, along the piston 20, from an terminal edge at the second end 12 of the housing 10, for sealing an interior space of the housing 10, which is illustrated in Fig.2 wherein the sealing member 30 extends outwards along the piston 20. By having the sealing member 30 extending inwards or outwards along the piston 20, from the housing 10 the risk of leakage from the housing 10 out into the surroundings is greatly reduced. Additionally, any leakage into the housing 10 is also reduced by the sealing member 30 extending outwards or inwards.

In an example, the implantable hydraulic displacement actuator 1 comprises a first fixation means 2. The first fixation means 2 is for anchoring the hydraulic actuator at an AV valve or valve plane, e.g. mitral valve and/or mitral valve plane such that the plane is moved with the movement of the actuator. The first fixation means is in an example at least one clamp, hook, barb, a suture, or any other fixation mean that is adapted to maintain the actuator at the mitral valve and/or AV-plane by squeezing, clamping, penetrating, pushing, pulling the actuator to the AV-plane or mitral valve and/or mitral valve plane and attach it there. This also includes any fixation means like a groove or a similar indentation or holes on or at the displacement actuator 1 that is adapted to have an ingrowth of tissue such as muscle tissue, and/or scar tissue in order to fixate or anchor the displacement actuator 1 to the mitral valve and/or mitral valve plane or AV-plane. Such fixation means is in an example detachable from the actuator 1 if for example an actuator 1 has to be changed while the first fixation means remain attached to the heart.

In an example the hydraulic actuator 1 is fixed arranged inside the muscle tissue of the heart. In an example the hydraulic actuator 1 is arranged partly inside the tissue of the heart, e.g. the hydraulic actuator 1 is angled outwards out from the heart or inwards into the atrium and/or ventricle. In an example the hydraulic actuator 1 is almost completely fixed inside the heart.

In an example, the implantable hydraulic displacement actuator 1 comprises a second fixation means 3 for anchoring the hydraulic actuator, such as the piston 20, at a distance from the AV planes, preferably on a ventricular side of an AV valve or valve plane. Thus the second fixation means might be fixated at or close to an apex of a heart or a heart ventricle, e.g inside a heart muscle, inside a heart muscle wall or on an outer surface of the heart. It may also be attached in adjacent non heart structures like the thoracic wall or the columna vertebrae (the spine). By having the displacement actuator 1 anchored at or close to the apex by the second anchor fixation 3 the displacement actuator 1 will be anchored at a distance which allows for the actuator to have enough stroke length to move an AV valve or valve plane, e.g. mitral valve and/or mitral valve plane a desired and/or required distance such that the pumping action of the heart is restored to normal function or improved close to normal function. Also, the ventricular walls of the heart comprises relatively thick heart muscles and is therefore a suitable location for the second fixation means 3 to be adapted to be anchored too. Additionally, the apex, and muscle adjacent to the apex, is capable of withstanding and counteracting the force from the displacement actuator 1 such that an AV valve or valve plane, e.g. the mitral valve and/or mitral valve plane is moved its desired length to restore normal pumping function of the heart. In an example, the piston 20 comprises an attachment element 50 at the second end 22 of the piston 20 for attaching second fixation means 3. By having the piston 20 comprising the attachment element 50 the piston 20 is easily secured to different objects. In an example the attachment element 50 couples to an anchor for attaching the displacement actuator 1 to the apex of the heart. Further, in an example the attachment element 50 has a releasable attachment mechanism in order to be able to detach the piston 20 when the displacement actuator 1 has to be exchanged or removed. Such releasable mechanism may in an example instead be located at the second fixation means 3 where it connects to the piston 20. In an example, the attachment element 50 comprises a thread, protrusion, clamp or the like for coupling to the anchor. In an example, the attachment element 50 is monolithic with the anchor for attaching to the object such as the apex of the heart or monolithic with the piston 20. By having the displacement actuator 1 anchored to an AV valve or valve plane, e.g. the mitral valve and/or mitral valve plane or the tricuspid valve or valve plane by means of the first fixation means, and to a second fixation point preferably on the ventricular side of the AV planes by means of the second fixation means, the displacement actuator 1 can move the mitral valve and/or mitral valve plane concurrently with the movement of the displacement actuator 1. Such actuator movement moves the housing 10 while the piston 20 is stationary.

In an example the displacement actuator 1 is a double acting actuator. By having the displacement actuator 1 being the double acting actuator the displacement actuator 1 is configured to produce a force in two directions, both on instroke and on outstroke of the piston 20 relatively to the housing 10. In an example, the two produced forces are applied to the mitral or tricuspid valve and/or mitral valve plane such that they move away or towards the apex of the heart, preferably along the long axis of the heart ventricle, for assisting the pumping function of the heart in both directions, towards the apex in systole and away from the apex in diastole. In an example the displacement actuator 1 is a single acting cylinder, thus producing a force in only one direction, on outstroke or on instroke of the piston 22 relatively to the housing 10. In an example, the single produced force moves the mitral valve and/or mitral valve plane away or toward the apex of the heart such that an assistance of the normal pumping function of the heart is in one direction only, either in systole or in diastole. The terms instroke and outstroke of the piston 20 in relation to its effect on the heart means that an outstroke of the piston 20 relative to the housing 10 will push the AV plane away from the apex of the heart. E.g. the mitral valve plane away from the second fixation means 3 which is attached on the ventricular side of the valve plane, thus it will assist the filling of a ventricle of a heart in diastole while the AV valve is open. On the other hand, an instroke of the piston 20 relative to the housing 10 will pull the housing 10 closer to the attachment 3. While doing this it will pull the AV plane closer to the apex, since the housing 10 is attached to the AV plane or valve annulus by means of the fixation means 2. Thus the movement will assist in a systole of the heart, augmenting the pump function of the heart as the AV plane itself works as a piston in the blood-stream while moving towards the apex when the AV valve is closed. In summary, it is the housing 10 that is moving up and down and the piston 20 is stationary. This movement of the hydraulic actuator 1 is contrary to other hydraulic devise or devices which squeeze the heart at the ventricular side at systole and thus counteract the natural AV-plane movement, thus worsen the natural pumping function.

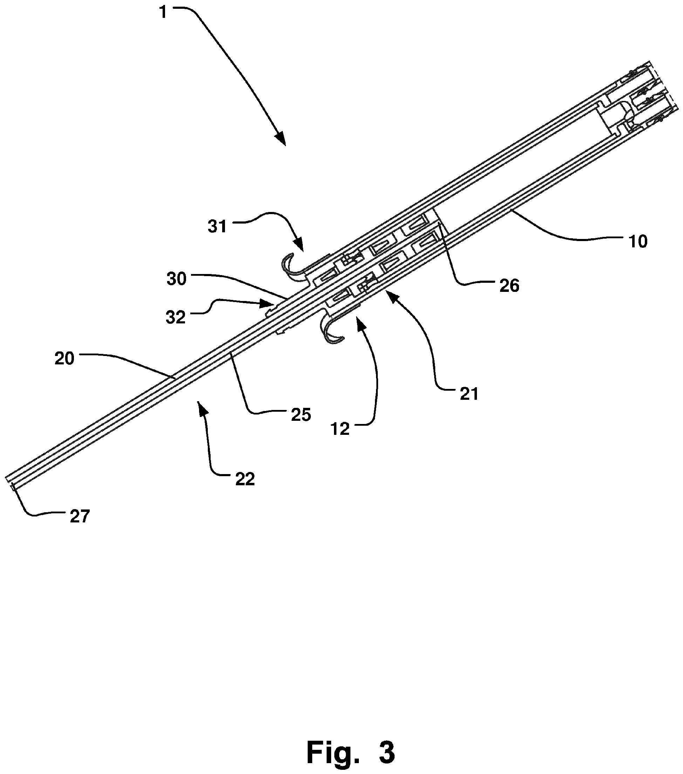

In an example, illustrated in e.g. fig.3, the first end 31 of the sealing member 30 is fixed on the housing 10 at the second end of the housing 10 and the second end 32 of the sealing member 30 is attached at a second end 22 of the piston 20 which is furthest away from the first end 11 of the housing 10, or attached at the first end 21 of the piston 20 inside the housing 10, such that the interior space of the housing 10 is sealed from the surroundings. By having the sealing member 30 being attached at the ends of the piston 20 the housing 10 and/or the piston 20. In an example when the sealing member 30 is attached to the end of the piston 20 which is furthest away from the first end 11 of the housing 10, a friction between the piston 20 and any tissue surrounding is reduced since the sealing member 30 will act as a friction reducing barrier arranged between the piston 20 and the surroundings. Further, in an example the sealing member 30 will prevent any adhesion of tissue, body fluids such as blood or the alike because it will be act as a protective barrier to the piston 20.

In an example, the sealing member 30 comprises an elastic and/or flexible material such as silicon, or other flexible polymers as for instance polyurethan. By having at least part of the sealing member 30 comprising flexible, the material like silicon of the sealing member 30 is adapted to withstand repeated movements of expansion and compression due to its elasticity and flexibility. Additionally, the friction between the piston 20 and any surrounding tissue is also reduced because of the low friction property of the sealing member 30 such as silicon. Further, the sealing member 30 such as silicon, acts as a protective barrier to the piston 20 from the surrounding tissue, which is illustrated in fig.3. Additionally, silicon, because of its elasticity, has a spring effect, which assists the piston 20 to move in the outstroke and/or instroke motion of the piston 20 of the actuator 1, depending of the piston 20 position.

In an example, the sealing member 30 comprises a plug element, and a compliant element. The plug element and the compliant element are attached to each other. The compliant element is the second end 32 of the sealing member 30, which extends outwards or inwards from the second end 12 of the housing 10, for sealing an interior space of the housing 10. By having the sealing member 30 comprising the plug element and the compliant element, the sealing member 30 can easily be constructed by separate components. Additionally, the use of separate elements provides the possibility to customise the elements dependent on their function as either the plug or the compliant element by e.g. choice of material, size, length, flexibility, sealing effect and so on. In an example, the plug element and the compliant element are made as a monolithic piece. By having the plug element and the compliant element made as a monolithic piece, the risk of any leakage is even further reduced since there are no surfaces between the plug element and conformable element where it can leak. Additionally, an easier manufacturing can be achieved by making the plug element and the compliant element as the monolithic piece.

In an example, the housing 10 comprises two chambers, an outer and an inner chamber. The piston 20 is partly slidably arranged in the inner chamber and the outer chamber encompasses the inner chamber such that a fluid is contained inside the two chambers making up the housing 10, as e.g. illustrated in fig.1.

In an example, the hydraulic displacement actuator 1 comprises means for disengaging the housing 10 from the piston 20, or vice versa, such that substantially no force is produced by the displacement actuator 1. By having means for disengaging the housing 10 from the piston 20 the displacement actuator 1 does not produce any force and thus the displacement actuator 1 moves together with and/or due to forces acting on the housing 10 and/or piston 20. In an example, the forces acting on the housing 10 and/or piston 20 are forces from a beating heart, wherein the displacement actuator 1 is arranged. In such an example, by having the displacement actuator 1 comprising means for disengaging the piston 20 and the housing 10, the displacement actuator 1 is free to move with the beating heart with a minimum or no counterforce at all. Such free movement is beneficial if the displacement actuator 1 would fail, when arranged inside the heart, because then the displacement actuator 1 is adapted to account for the failure and capable of being inside the heart but without substantially affecting the heart and possible damaging the heart. Hence, the biocompatible hydraulic fluid can freely flow in or at the housing 10 and/or between the housing 10 and the piston 20, such as between the two chambers, and hence does not hinder movement and/or contribute to movement of the piston 20 and/or housing 10. In turn, the heart is substantially not hindered in its movement by the implanted displacement actuator 1. In an example, the disengaging is used even though the actuator is not failing e.g. in order to reduce energy consumption. Such energy consumption decoupling is an example used when assistance of heart function is not necessary, for instance when resting or during sleep. Such decoupling is in an example performed in sequence with the heart beats, for example decoupling during every other heart beat or every third heart beat or any other rhythm pattern.

In an example the disengaging means is arranged at the first end 21 of the piston 20 such that the disengaging means is arranged at a head of the piston 20 which is fluidly arranged between the two chambers, and thus is configured to disengage the two chambers by controlling the flow between the chambers. By having the disengaging means arranged at the head of the piston 20 a minimum friction of flow of the fluid is accomplished. In an example, the disengaging means is arranged at other locations in the hydraulic displacement actuator 1 such that an open fluid flow loop is created for fluid to flow in the hydraulic displacement actuator 1 without producing any force and/or movement of the housing 10 or piston 20.

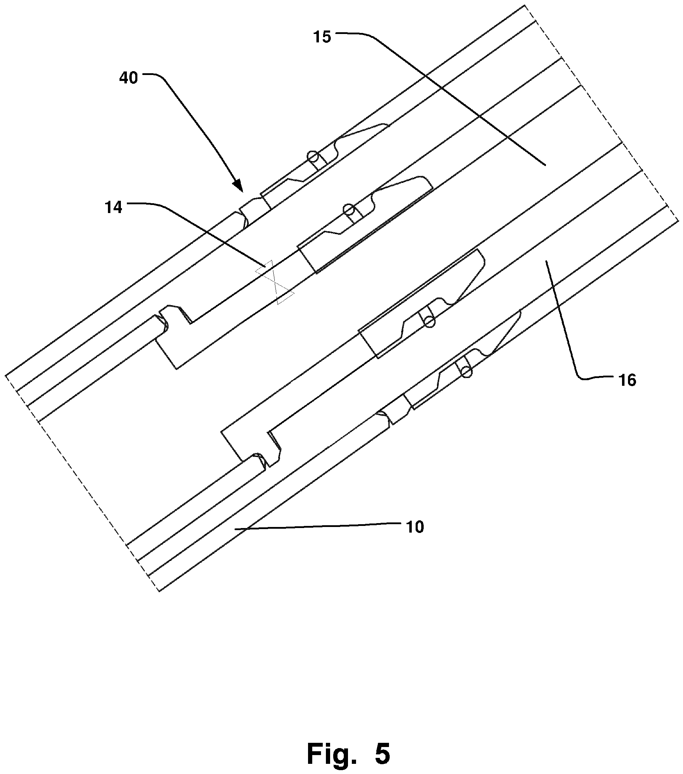

In an example, the means for disengaging is a valve 14, as illustrated in fig.5. By having the valve 14 a differential pressure between the housing 10 and the piston 20 is equalized when the valve is open, and the housing 10 and the piston 20 can move freely. In an example, the valve 14 is configured to be open at a default state thus allowing for free flow and substantially no force to be generated by the displacement actuator 1. In a working state the valve 14 is closed such that the displacement actuator 1 generates a force and the flow of fluid is restricted such that it acts on the piston 20. By having this configuration of default state and working state the hydraulic actuator 1 saves energy if the valve 14 is controlled by energy such as electricity, pressure, force or the like.

In an example, the valve 14 is arranged between the hydraulic fluid inlet-outlet tube 15 and the inlet-outlet tube 16 adapted for pressurizing the housing 10 differentially. By having the valve 14 arranged between the tubes 15 and 16 the pressure is easily equalised on both sides of the piston 20 in the housing 10. In an example the valve 14 is arranged at the head of the piston 20, giving a same effect as described above. In an example, the valve 14 is a gate valve, check valve, a ball valve or any other known valve used for directing fluids and/or gases and configured to be controlled.

In an example, the piston 20 comprises a fluid channel 25, as e.g. illustrated in figs. 2 and 3. The fluid channel 25 has a first opening 26 and extends from the first end 21 of the piston 20, closest to the first end 11 of the housing 10, and to a second opening 27 at the extending section 22 of the piston 20. In an example, the second opening 27 is arranged at the end of the piston 20 such that a guide wire is insertable through the hydraulic displacement device 1 from the first end 11 of the housing 10 and inside the housing 10 then going through the first opening 26 through fluid channel 25 and out through the second opening 27 to the outside of the hydraulic displacement device 1. By having this free passageway through the hydraulic displacement device 1 the hydraulic displacement device 1 is easily guided by use of the guide wire. Thus a guide wire may be placed through the whole hydraulic actuator 1, from the second fixation means 3, through the attachment means 50, through the piston 20, further through the housing 10 and further out into the inlet-outlet tube 15 or 16. Inlet-outlet tubes 15 and 16 are in an example at least temporarily extended through blood vessels until outside of the body. An operator may thus from outside the body thread the displacement actuator 1 over the guide wire and push the displacement actuator 1 all the way from outside the body with the attachment means 50 for connection to the second fixation means 3. In an example the fixation means 3 may previously have been attached in the heart, in preparation for an attachment. Further, in another example, the first fixation means 2 is released and attached to the AV plane structures substantially simultaneously as the connection to the second fixation means 3 occurs. In an example, these two attachments are performed at different times. Preferably the device is inserted over a guide wire when positioned inside a delivery catheter. The guide wire can be detached from the second fixation means 3 and retracted from the displacement actuator 1 when further not needed, or at least partly left in place. In an example the guide wire is detachable attached to the attachment means 50.

In an example, the second opening 27 is arranged perpendicular to an extension of the piston 20, as e.g. illustrated in fig 2. By having the second opening 27 arranged sideways outwards from the extending section 22 of the piston 20, a contact surface between the extending section 22 of the piston 20 and the sealing member 30 is reduced since the displacement fluid enters in between the sealing member 30 and the extending section 22 of the piston 20 through the openings 26 and 27 and thus reduces friction between the sealing member 30 and the extending section 22 of the piston 20. Additionally, the fluid channel 25 together with second openings 27 arranged sideways and outwards, allows for pressurizing the sealing member 30 such that the sealing member 30 will move and thus further prevent tissue to adhere or grow to the sealing member 30.

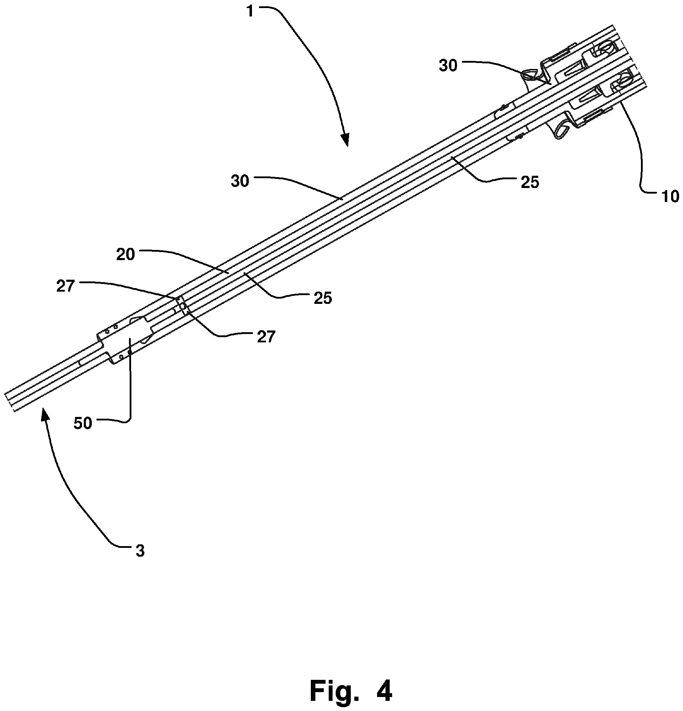

In an example, the opening 27 at the extending section 22 comprises a plurality of openings, as illustrated in fig.4. By having a plurality of openings 27 at the extending section 22 the displacement fluid is diverted in between the sealing member 30 and the extending section 22 of the piston 20 in a more even way. This will lubricate and lower any friction there between in a more uniform manner. Further, this will create a more uniform movement of the sealing member 30 for reducing ingrowth.

In an example, a pressure in the housing 10 or on the piston 20 is in a range of 2-100bar, more specifically 30-40bar, or even more specifically, 1-10bar. The force of the hydraulic actuator 1 is calculated with the equations:

The force produced by a double acting hydraulic piston on the rod side can be expressed as:

$$F1 = (pi*(d2^2-d1^2)/4)*P1,$$

where

F1 = rod pull force
d1 = rod diameter
d2 = piston diameter
P1 = pressure in the cylinder (differential pressure)

The force produced opposite the rod can be expressed as:

$$F2 = (pi*d2^2/4)*P2,$$

where

F2 = rod push force
P2 = pressure in the cylinder (differential pressure)

In an example of a rod with a diameter of 4mm and a piston with a diameter of 2mm working at 6 bar will produce forces a of pull force 7,6N and a push force 5,6N. In an example as above but with a working pressure of 4 bar will produce forces of 5N and 3,8N. In an example as above but with a working pressure of 2 bar will produce forces of 2,5N and 1,9N.

In an example, the hydraulic displacement actuator 1 comprises a combined inlet and outlet element 40 which is attached at the first end 11 of the housing 10. By having a combined inlet and outlet element 40 at the first end 11 of the housing 10 the overall size of the displacement actuator 1 can be kept small and compact. In an example, when the displacement actuator 1 having the small and compact design is arranged inside the heart there is avoided any protruding parts to the sides out from the displacement actuator 1 which thus reduces the risk of damaging the heart surrounding the displacement actuator 1. Further, in an example the combined inlet and outlet element 40 makes it possible to configure the displacement actuator 1 in a streamlined manner together with design of the housing 10 comprising two chambers and which the combined inlet and outlet 40 connects to. By streamlining the displacement actuator 1 any disturbance to the natural flow of the blood in the heart when passing the displacement actuator 1 is reduced. Additionally, the streamlined design prohibits any substances to easily get caught on the displacement actuator 1 and lead to complications within the heart such as clothing, tissue ingrowth or other unwanted cardiac complications. Furthermore, the streamlined design permit the actuator 1 to easily be transferred through a catheter with minimal friction in order to be able to deliver the actuator 1 and the fixation means 2,3 to the inside structures of the heart.

In an example, e.g. illustrated in fig.5 the combined inlet and outlet element 40 is arranged such that inlet-outlet tubes 15 and 16 are concentric for an even easier and slimmer construction of the hydraulic actuator 1.

In an example, the piston 20 is stationary, while the housing 10 is moving while acting. By having the piston 20 stationary and moving the housing 10 instead of moving the piston as traditionally is done, the housing 10, which is the largest element of the piston 20 and the housing 10, can be arranged such that a greatest supporting surface of the piston 20 and the housing 10 is in contact with a desired object to be moved by the displacement actuator 1. In an example, the housing 10 is attached to the mitral valve(s) and/or mitral valve plane or to the tricuspid valve and/or tricuspid valve plane within the heart. This allows, when the actuator 1 is in action, for maximum assistance to the pump function of the heart and thereby increasing the blood flow through the heart wherein the displacement actuator 1 is arranged, since the housing 10 is arranged adjacent AV valves, and thus is capable of move and/or support AV valves, e.g. the mitral valve movement.

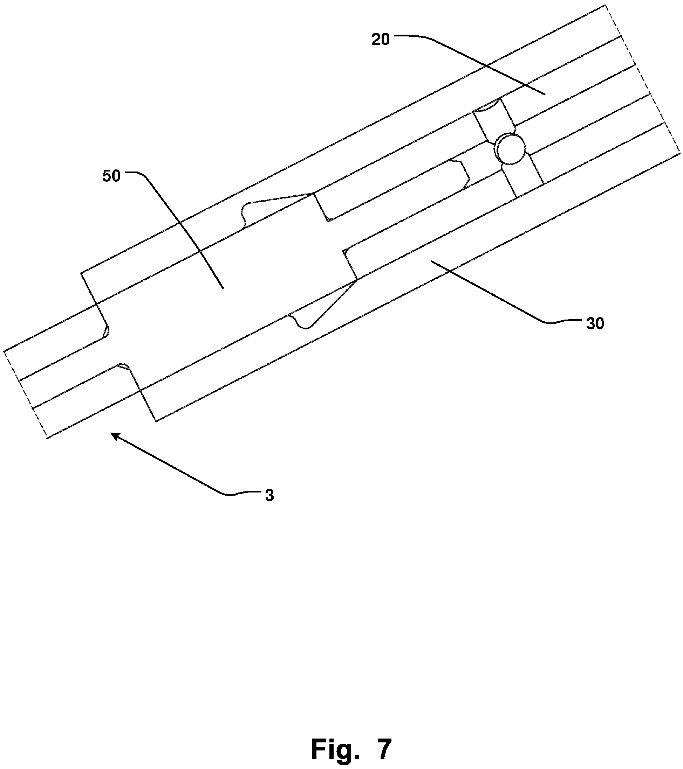

In an example, a system 100 for moving a hydraulic displacement actuator, illustrated in e.g. fig.7, comprises a hydraulic displacement actuator 1 and a pressurizing means 60 connected to the hydraulic displacement actuator for pressurizing a biocompatible hydraulic displacement fluid comprised in the hydraulic displacement actuator 1. The displacement actuator 1 comprises a housing 10, comprising a first end 11 and a second end 12, a piston 20, comprising a first end 21 and an extending section 22, wherein the first end 21 of the piston 20 is arranged in the housing 10 and the extending section 22 is arranged at least partly outside the housing 10.

In an example, the displacement actuator 1 comprises a sealing member 30, comprising a first end 31 and a second end 32, and wherein the first end 31 of the sealing member 30 is secured to the second end 12 of the housing 10 and the second end 32 of the sealing member 30 extends outwards or inwards, along the piston 10, from a terminal edge at the second end 12 of the housing 10, for sealing an interior space of the housing 10. By having the system 100 comprising the displacement actuator 1, comprising the biocompatible hydraulic displacement fluid and the pressurizing means 60, the displacement actuator 1 is controllably moved and the force by which the displacement actuator 1 is moved is determined by the pressurizing means 60.

In an example, the system 100 comprises a control unit connected to the pressurizing means 60, and at least one sensor, and in communication with the control unit. By using the at least one sensor and the control unit, the control unit is configured to control the hydraulic actuator 1 based on sensor information from the at least one sensor.

In an example, the at least one sensor is a pressure sensor, position sensor, accelerometer, velocity sensor, electrical sensor, or other sensor used for measuring cardiac parameters.

In an example the system 100 comprises at least one sensor adapted to detect an electrical signal e. g electrocardiogram (ECG) of the heart, and in communication with the control unit. By having the system 100 comprising at least one sensor adapted to detect an electrical signal of the heart and in communication with the control unit, the control unit can control the pressurizing means 60 by use of the electrical signal of the heart e.g. the beat of the heart, in synchronicity with the existing heart cycle. In an example, the control unit controls the pressurizing means 60 in response to electrical signals of the heart for diastole and/or systole.

In another example the system 100 comprises at least one pressure sensor adapted to detect a pressure inside a heart cavity of the heart, outside the actuator 1, and in communication with the control unit. By having the system 100 comprising at least one sensor adapted to detect a pressure signal of the heart and in communication with the control unit, the control unit can control the pressurizing means 60 by use of the pressure signal inside the heart e.g. left ventricular or atrial pressure, in synchronicity with the existing heart cycle.

In an example, the control unit controls the pressurizing means 60 in response to pressure signals of the heart for diastole and/or systole.

Furthermore, a pressure sensor inside the heart may be used to regulate necessary actuator effect over a longer time span than a beat-to-beat cycle, e.g. hours to days, especially in order to household with energy.

In an example, the system 100 comprises at least one pressure sensor adapted to detect a pressure of the hydraulic displacement actuator 1. Such pressure sensor allows for supervision and guidance of pressure patterns inside the actuator 1.

In an example the system 100 comprises at least one sensor adapted to detect position signals and being in communication with the control unit. By having the system 100 comprising at least one sensor adapted to detect at least one position signal of the heart and or parts of the actuator system and in communication with the control unit, the control unit can control the pressurizing means 60 by use of the position signal in synchronicity with the existing heart cycle and or actuator action. In an example, the control unit controls the pressurizing means 60 in response to position signals of the heart for diastole and/or systole.

Further an example of the system 100 comprises at least one sensor adapted to detect an acceleration signal, and in communication with the control unit. By having the system 100 comprising at least one sensor adapted to detect acceleration of parts of the heart e.g. a valve plane, and or parts of the actuator system and in communication with the control unit, the control unit can control the pressurizing means 60 by use of the acceleration signal of the heart e.g. the beat of the heart, and the action of the actuator 1 is in synchronicity with the existing heart cycle. In an example, the control unit controls the pressurizing means 60 in response to acceleration signals of the heart for diastole and/or systole.

Further an example of the system 100 comprises at least one sensor adapted to detect a volume signal, and in communication with the control unit. By having the system 100 comprising at least one sensor adapted to detect volumes, in the whole or parts of the actuator system and in communication with the control unit, the control unit can control the biocompatible hydraulic fluid management of the system 100.

In an example the pressurizing means 60 comprises means for refilling the biocompatible hydraulic displacement fluid. By having the pressurizing means comprising means for refilling the biocompatible fluid it is possible to refill the system if fluid has leaked out from the displacement actuator 1. In an example the means for refilling is a membrane that can be punctured by a needle or the like and a reservoir so that the system can be refilled by simply puncturing the membrane and injecting the biocompatible fluid into the system 100. Such refill may be done through the skin of a patient by means of a needle for instance. In an example the refill is performed at the hydraulic displacement actuator 1.

In an example the system 100 comprises a battery for providing power. In an example the battery is implanted together with the hydraulic actuator or in another example remote to that location.

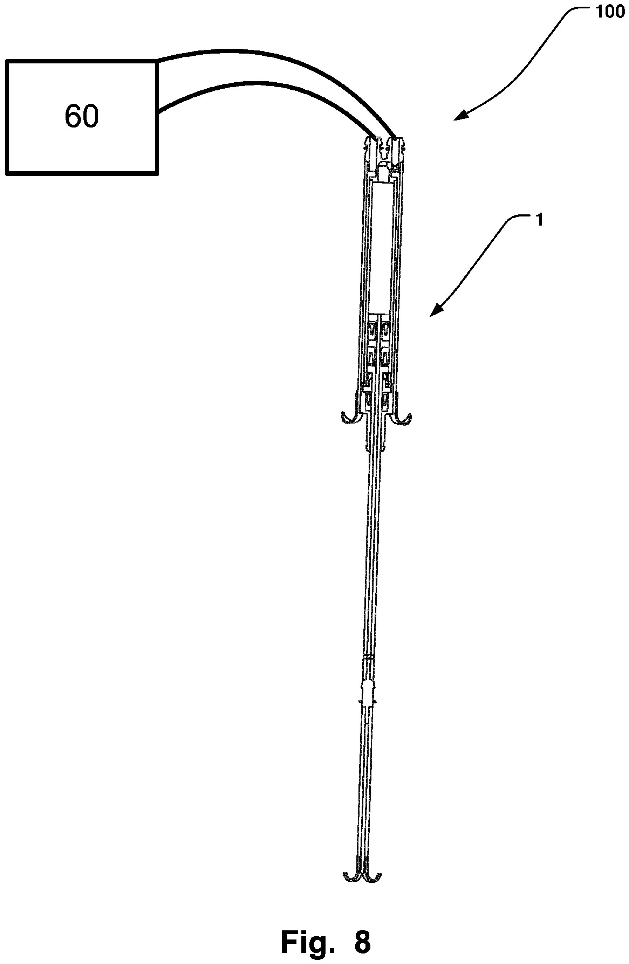

In an example, an exemplary method of displacing a hydraulic displacement actuator 1, illustrated in fig. 8, is disclosed which comprises, providing 110 the hydraulic displacement actuator 1 and moving 120 a housing 10 in a first direction by pressurizing a first space, comprising the biocompatible hydraulic displacement fluid, arranged between a first end 11 of the housing 10 and a first end 21 of a piston 20, or by moving the housing 10 in an opposite direction from the first direction by pressurizing a second space, comprising the biocompatible hydraulic displacement fluid, arranged between a second end 12 of the housing 10 and the first end 21 of the piston 20. The hydraulic displacement actuator 1 comprises the housing 10 which comprises the first end 11 and a second end 21, the piston 20, comprising the first end 21 and an extending section 22, wherein the first end 21 of the piston 20 is arranged in the housing 10 and the extending section 22 is arranged at least partly outside the housing 10. In an example, the displacement actuator 1 also comprises a sealing member 30, comprising a first end 31 and a second end 32, and wherein the first end 31 of the sealing member 30 is secured to the second end 32 of the housing 10 and the second end 32 of the sealing member 30 extends outwards or inwards, along the piston 20, from a terminal edge at the second end of the housing 10, for sealing an interior space of the housing 10. By pressurizing the displacement actuator 1 on either side of the piston 20 the displacement actuator 1 will move and produce a force, which moves the housing 10 for moving the mitral valve or the mitral valve plane or the tricuspid valve or the tricuspid valve plane. The movement of the displacement actuator 1 will thus assist heart pump function either in diastole or systole or parts of systole or parts of diastole, or both. In an example, the displacing of the hydraulic displacement actuator 1 comprises pressurizing both first and second spaces(chambers) in sequence such that a double acting movement of the housing 10 is produced. By pressurizing the biocompatible hydraulic displacement fluid on both sides of the piston 20 alternately the displacement actuator 1 is a double acting actuator that can generate force in two directions and thus assist in both diastole and systole. In an example, the directions are substantially in a direction of an extension of the hydraulic displacement actuator 1. Preferably such action is along the long axis heart ventricles from the AV valves towards the heart apex along the natural blood flow direction

In an example, the exemplary method comprises providing a piston 20 comprising a fluid channel 25 having a first opening 26 and extending from a first end 21 of the piston 20, closest to the first end 11 of the housing 10, and to a second opening 27 at the extending section 22 of the piston 20. Such channel 25 had double functions, primarily for fluid passage, but secondly for passing a guide wire during device implant. Additionally, a contact surface between the extending section 22 of the piston 20 and the sealing member 30 will be lubricated by the pressurised fluid being pushed in between the piston 20 and the sealing member 30.

In an example, a non-transitory medium having instructions for 3D-printing the hydraulic actuator 1 is disclosed. By having instructions of 3D printing the actuator 1 it is possible to use a 3D printer for printing e.g. tailored or non-tailored implantable hydraulic actuators 1. The hydraulic actuators 1 is in an example tailored after the patients bio data such as, size of the heart, thickness of the heart and other relevant cardiac features.

The present disclosure has been described above with reference to specific examples. However, other examples than the above described are equally possible within the scope of the disclosure. Different exemplary method steps or a different order thereof than those described above may be provided within the scope of the disclosure. The different features and steps of the disclosure may be combined in other combinations than those described. The scope of the invention is only limited by the appended patent claims.

## Claims

1.  An implantable hydraulic displacement actuator (1) comprising a biocompatible hydraulic displacement fluid for providing a force inside a human or animal body, **characterized in that** said hydraulic displacement actuator comprises:

    - a first fixation means (2) for anchoring the hydraulic displacement actuator at an AV valve or valve plane; and

- a second fixation means (3) for anchoring the hydraulic displacement actuator at a distance from an AV valve or plane adjacent an apex of a heart and/or in a heart muscle, where said hydraulic displacement actuator is a linear hydraulic actuator for generating a cardiac movement.

2. A hydraulic displacement actuator according to claim 1 comprising:

   - a housing (10), comprising a first end (11) and a second end (12),
   - a piston (20), comprising a first end (21) and an extending section (22), wherein the first end of the piston is arranged in the housing and the extending section is arranged at least partly outside the housing,
   - a sealing member (30), comprising a first end (31) and a second end (32), and wherein the first end of the sealing member is secured to the second end of the housing and the second end of the sealing member extends outwards or inwards, along the piston, from a terminal edge at the second end of the housing, for sealing an interior space of the housing.

3. A hydraulic displacement actuator according to claim 2, wherein the hydraulic displacement actuator is a double acting actuator.

4. A hydraulic displacement actuator according to any of claims 2-3, wherein the first end of the sealing member is attached at the housing at the second end of the housing, and the second end of the sealing member is attached at a second end of the piston which is furthest away from the first end of the housing, or attached at the first end of the piston inside the housing, such that the interior space of the housing is sealed from its surroundings; and/or wherein the sealing member comprises flexible silicon.

5. A hydraulic displacement actuator according to any of claims 2-4, wherein the sealing member comprises:

   - a plug element, and
   - a compliant element, which are attached to each other and the compliant element is the second end of the sealing member, which extends outwards or inwards from the second end of the housing, for sealing an interior space of the housing, and wherein the plug element and the compliant element optionally are made as a monolithic piece.

6. A hydraulic displacement actuator according to any of claims 2 to 5, wherein the hydraulic displacement actuator comprises means for disengaging the housing from the piston, or vice versa, such that the hydraulic fluid flows freely and substantially no force is produced by the displacement actuator.

7. A hydraulic displacement actuator according to claim 6, wherein the housing comprises two chambers, an outer chamber and an inner chamber, wherein the inner chamber is arranged in the outer chamber, and the means for disengaging is arranged between the inner and outer chamber such that the hydraulic fluid runs freely between the chambers.

8. A hydraulic displacement actuator according to claim 6 or 7, wherein the means for disengaging is a valve, and wherein the valve optionally is arranged between an inlet tube and an outlet tube for pressurizing the housing.

9. A hydraulic displacement actuator according to any of claims 2 to 8, wherein the piston comprises a fluid channel having a first opening and extending from a first end of the piston, closest to the first end of the housing, and to a second opening at the extending section of the piston, and wherein the opening at the extending section preferably comprises a plurality of openings.

10. A hydraulic displacement actuator according to any claims 2 to 9, wherein a pressure on the piston is in a range of 2-100bar, more specifically 30-40bar, or even more specifically, 1-10bar; and/or wherein the hydraulic displacement actuator comprises a combined inlet and outlet element that is attached at the first end of the housing; and/or wherein the piston is stationary and the housing is moving in an inwards and outwards motion relative to the piston.

11. A system (100) for moving an implantable hydraulic displacement actuator **characterised in that** the system comprises:

   - a hydraulic displacement actuator according to any preceding claim; and
   - a pressurizing means (60) for pressurizing the biocompatible hydraulic displacement fluid and the hydraulic displacement actuator.

12. A system according to claim 11, wherein the system comprises:

   - a control unit connected to the pressurizing means, and
   - at least one sensor in communication with the control unit; and/or comprising at least one sensor adapted to detect an electrical signal of the heart, or a pressure of the heart, and in communication with the control unit; and/or wherein the pressurizing means comprises means for refilling the biocompatible hydraulic displacement fluid.

**Patentansprüche**

1. Ein implantierbarer hydraulischer Verdrängungsaktuator (1) mit einer biokompatiblen Verdrängungsflüssigkeit zur Bereitstellung einer Kraft in einem menschlichen oder tierischen Körper, **dadurch gekennzeichnet, dass** der vorerwähnte hydraulische Verdrängungsaktuator Folgendes aufweist:

   - ein erstes Befestigungsmittel (2) zur Verankerung des hydraulischen Verdrängungsaktuators an einer atrioventrikulären Klappe oder einer Klappenebene und
   - ein zweites Befestigungsmittel (3) zur Verankerung des hydraulischen Verdrängungsaktuators in einer gewissen Distanz von der atrioventrikulären Klappe oder Ebene angrenzend an eine Herzspitze und/oder in einem Herzmuskel, wobei es sich bei dem vorerwähnten implantierbaren hydraulischen Verdrängungsaktuator um einen linearen hydraulischen Aktuator zur Erzeugung einer Herzbewegung handelt.

2. Ein hydraulischer Verdrängungsaktuator gemäß Anspruch 1 mit:

   - einem Gehäuse (10) mit einem ersten Ende (11) und einem zweiten Ende (12),
   - einem Kolben (20) mit einem ersten Ende (21) und einem Verlängerungsbereich (22), wobei das erste Ende des Kolbens im Gehäuse angeordnet ist und der Verlängerungsbereich zumindest teilweise außerhalb des Gehäuses angeordnet ist,
   - einem Dichtelement (30) mit einem ersten Ende (31) und einem zweiten Ende (32), wobei das erste Ende des Dichtelements am zweiten Ende des Gehäuses gesichert ist und das zweite Ende des Dichtelements außen oder innen entlang des Kolbens von einer Abschlusskante am zweiten Ende des Gehäuses verläuft, um so einen Innenraum des Gehäuses abzudichten.

3. Ein hydraulischer Verdrängungsaktuator gemäß Anspruch 2, wobei es sich bei dem hydraulischen Verdrängungsaktuator um einen doppeltwirkenden Aktuator handelt.

4. Ein hydraulischer Verdrängungsaktuator gemäß einem der Ansprüche 2-3, wobei das erste Ende des Dichtelements am zweiten Ende des Gehäuses und das zweite Ende des Dichtelements am zweiten, am weitesten vom ersten Ende des Gehäuses entfernten Ende des Kolbens oder am ersten Ende des Kolbens innerhalb des Gehäuses befestigt ist, so dass der Innenraum des Gehäuses von der Umgebung abgedichtet ist und/oder wobei das Dichtelement einen Silikonschlauch aufweist.

5. Ein hydraulischer Verdrängungsaktuator gemäß einem der Ansprüche 2-4, wobei das Dichtelement Folgendes aufweist:

   - ein Steckelement und
   - ein Gegenstück,
   die miteinander verbunden werden, wobei das Gegenstück das zweite Ende des Dichtelements ist, das sich außerhalb oder innerhalb vom zweiten Ende des Gehäuses erstreckt, um einen Innenraum des Gehäuses abzudichten, und wobei das Steckelement und das Gegenstück optional monolithisch ausgeführt sind.

6. Ein hydraulischer Verdrängungsaktuator gemäß einem der Ansprüche 2-5, wobei der hydraulische Verdrängungsaktuator ein Mittel zur Freigabe des Gehäuses vom Kolben oder umgekehrt aufweist, so dass die Hydraulikflüssigkeit frei fließt und im Wesentlichen keine Kraft vom Verdrängungsaktuator aufgebracht wird.

7. Ein hydraulischer Verdrängungsaktuator gemäß Anspruch 6, wobei das Gehäuse zwei Kammern aufweist, eine äußere und eine innere, wobei die innere Kammer in der äußeren Kammer angeordnet ist, und das Mittel zur Freigabe zwischen innerer und äußerer Kammer angeordnet ist, so dass die Hydraulikflüssigkeit frei zwischen den Kammern zirkulieren kann.

8. Ein hydraulischer Verdrängungsaktuator gemäß Anspruch 6 oder 7, wobei das Mittel zur Freigabe ein Ventil ist und wobei das Ventil optional zwischen einem Einlassrohr und einem Auslassrohr zur Druckbeaufschlagung des Gehäuses angeordnet ist.

9. Ein hydraulischer Verdrängungsaktuator gemäß einem der Ansprüche 2-8, wobei der Kolben einen Flüssigkeitskanal mit einer ersten Öffnung aufweist und sich von einem ersten Ende des Kolbens, das am nächsten zum ersten Ende des Gehäuses liegt, bis zu einer zweiten Öffnung im Verlängerungsbereich des Kolbens erstreckt, wobei die Öffnung im Verlängerungsbereich vorzugsweise eine Mehrzahl von Öffnungen aufweist.

10. Ein hydraulischer Verdrängungsaktuator gemäß einem der Ansprüche 2-9, wobei der Druck auf dem Kolben im Bereich 2-100 bar liegt, spezieller 30-40 bar oder noch spezieller 1-10 bar und/oder wobei der hydraulische Verdrängungsaktuator ein kombiniertes Ein- und Auslasselement enthält, das am ersten Ende des Gehäuses befestigt ist und/oder wobei der Kolben stationär ist und das Gehäuse eine in Bezug auf den Kolben relative Einwärts- und Auswärtsbewegung beschreibt.

11. Ein System (100) zur Bewegung eines implantierbaren hydraulischen Verdrängungsaktuators, **dadurch gekennzeichnet, dass** das System Folgendes umfasst:

- einen hydraulischen Verdrängungsaktuator gemäß einem der vorstehenden Ansprüche und
- ein Druckbeaufschlagungsmittel (60) zur Druckbeaufschlagung des biokompatiblen hydraulischen Verdrängungsaktuators und den hydraulischen Verdrängungsaktuator.

12. Das System gemäß Anspruch 11, wobei das System weiterhin Folgendes beinhaltet:

- eine an das Druckbeaufschlagungsmittel angeschlossene Steuereinheit und
- mindestens einen mit der Steuereinheit kommunizierenden Sensor und/oder mindestens einen Sensor, der zur Feststellung eines elektrischen Herzsignals oder eines Blutdrucks ausgelegt ist und mit der Steuereinheit in Verbindung steht und/oder
wobei das Druckbeaufschlagungsmittel Mittel zum Nachfüllen der biokompatiblen Verdrängungsflüssigkeit aufweist.

## Revendications

1. Actionneur de déplacement hydraulique implantable (1) comprenant un fluide de déplacement hydraulique biocompatible destiné à délivrer une force à l'intérieur d'un corps humain ou animal, **caractérisé en ce que** ledit actionneur de déplacement hydraulique comprend :

- un premier moyen de fixation (2) destiné à ancrer l'actionneur de déplacement hydraulique au niveau d'une valvule auriculoventriculaire (valvule AV) ou d'un plan de valvule AV ; et
- un deuxième moyen de fixation (3) destiné à ancrer l'actionneur de déplacement hydraulique à une certaine distance d'une valvule AV ou d'un plan de valvule AV de manière adjacente à un apex d'un cœur et/ou dans un muscle cardiaque,
où ledit actionneur de déplacement hydraulique est un actionneur hydraulique linéaire destiné à générer un mouvement cardiaque.

2. Actionneur de déplacement hydraulique selon la revendication 1, comprenant :

- un logement (10), comprenant une première extrémité (11) et une seconde extrémité (12),
- un piston (20), comprenant une première extrémité (21) et une section d'extension (22), où la première extrémité du piston est agencée dans le logement et la section d'extension est agencée au moins en partie en dehors du logement,
- un élément d'étanchéité (30), comprenant une première extrémité (31) et une seconde extrémité (32), et
où la première extrémité de l'élément d'étanchéité est fixée sur la seconde extrémité du logement et la seconde extrémité de l'élément d'étanchéité s'étend vers l'extérieur ou vers l'intérieur, le long du piston, depuis un bord terminal au niveau de la seconde extrémité du logement, afin de fermer hermétiquement un espace intérieur du logement.

3. Actionneur de déplacement hydraulique selon la revendication 2, où l'actionneur de déplacement hydraulique est un actionneur à double action.

4. Actionneur de déplacement hydraulique selon l'une quelconque des revendications 2 et 3, dans lequel la première extrémité de l'élément d'étanchéité est attachée au niveau du logement au niveau de la seconde extrémité du logement, et la seconde extrémité de l'élément d'étanchéité est attachée au niveau d'une seconde extrémité du piston, laquelle est la plus éloignée de la première extrémité du logement, ou attachée au niveau de la première extrémité du piston à l'intérieur du logement, de telle sorte que l'espace intérieur du logement est fermé de manière hermétique par rapport à son environnement ; et/ou où l'élément d'étanchéité comprend du silicone flexible.

5. Actionneur de déplacement hydraulique selon l'une quelconque des revendications 2 à 4, dans lequel l'élément d'étanchéité comprend :

- un élément de plongeur, et
- un élément souple,
lesquels sont attachés l'un à l'autre et l'élément souple est la seconde extrémité de l'élément d'étanchéité, laquelle s'étend vers l'extérieur ou vers l'intérieur depuis la seconde extrémité du logement, afin de fermer de manière hermétique un espace intérieur du logement, et où l'élément de plongeur et l'élément souple optionnellement sont réalisés sous la forme d'une pièce monolithique.

6. Actionneur de déplacement hydraulique selon l'une quelconque des revendications 2 à 5, où l'actionneur de déplacement hydraulique comprend un moyen destiné à désengager le logement du piston, ou vice versa, de telle sorte que le fluide hydraulique s'écoule librement et que sensiblement aucune force n'est produite par l'actionneur de déplacement.

7. Actionneur de déplacement hydraulique selon la revendication 6, dans lequel le logement comprend deux chambres, une chambre extérieure et une chambre intérieure, où la chambre intérieure est agencée dans la chambre extérieure, et le moyen de désengagement est agencé entre les chambres intérieure et extérieure de telle sorte que le fluide hydraulique circule librement entre les chambres.

8. Actionneur de déplacement hydraulique selon la revendication 6 ou 7, dans lequel le moyen de désengagement est une valve, et où la valve optionnellement est agencée entre un tube d'entrée et un tube de sortie afin de mettre en pression le logement.

9. Actionneur de déplacement hydraulique selon l'une quelconque des revendications 2 à 8, dans lequel le piston comprend un canal de fluide présentant une première ouverture et s'étendant depuis une première extrémité du piston, la plus proche de la première extrémité du logement, et jusqu'à une seconde ouverture au niveau de la section d'extension du piston, et où l'ouverture au niveau de la section d'extension de préférence comprend une pluralité d'ouvertures.

10. Actionneur de déplacement hydraulique selon l'une quelconque des revendications 2 à 9, dans lequel une pression sur le piston se situe dans une plage allant de 2 à 100 bar, de façon plus spécifique de 30 à 40 bar, ou de façon encore plus spécifique de 1 à 10 bar ; et/ou où l'actionneur de déplacement hydraulique comprend un élément d'entrée et de sortie combinées qui est attaché à la première extrémité du logement ; et/ou où le piston est stationnaire et le logement est mobile selon un mouvement vers l'intérieur et vers l'extérieur par rapport au piston.

11. Système (100) destiné à déplacer un actionneur de déplacement hydraulique implantable **caractérisé en ce que** le système comprend :

- un actionneur de déplacement hydraulique selon l'une quelconque des revendications précédentes ; et

- un moyen de mise en pression (60) destiné à mettre en pression le fluide de déplacement hydraulique biocompatible et l'actionneur de déplacement hydraulique.

12. Système selon la revendication 11, où le système comprend :

- une unité de commande reliée au moyen de mise en pression, et
- au moins un capteur en communication avec l'unité de commande ; et/ou comprenant au moins un capteur conçu pour détecter un signal électrique du cœur, ou une pression du cœur, et en communication avec l'unité de commande ; et/ou où le moyen de mise en pression comprend un moyen destiné à assurer un re-remplissage en fluide de déplacement hydraulique biocompatible.

**Fig. 1**

# Fig. 2

**Fig. 3**

**Fig. 4**

**Fig. 5**

Fig. 6

**Fig. 7**

**Fig. 8**

```
┌─────────────────┐
│                 │
│      110        │
│                 │
└────────┬────────┘
         │
┌────────┴────────┐
│                 │
│      120        │
│                 │
└─────────────────┘
```

# Fig. 9

**EP 3 237 033 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004153067 A1
- US 2006178550 A1